# EUROPEAN PATENT APPLICATION

(11) **EP 4 060 332 A1**
(43) Date of publication of application: **21.09.2022**
(21) Application number: 20887058.4
(22) Date of filing: 22.10.2020
(51) Int. Cl.: G01N 27/447, C12Q 1/68

(54) **HORIZONTAL ELECTROPHORESIS APPARATUS**

(30) Priority: 12.11.2019 KR 20190144489
(71) Applicant: T-Mac Co., Ltd., Daejeon 34054 (KR); Korea Research Institute of Bioscience and Biotechnology, Daejeon 34141 (KR)
(72) Inventor: KANG, Sung Hyun, Daejeon 34140 (KR); PARK, Jong Taek, Daejeon 34032 (KR); LIM, Dong Woo, Sejong-si 30064 (KR); HA, Woo Jong, Sejong-si 30063 (KR); SHIN, Heung Seon, Sejong-si 30098 (KR)
(74) Representative: Keltie LLP
(86) International application number: PCT/KR2020/014508
(87) International publication number: WO 2021/096092

(57) **Abstract**

A horizontal electrophoresis apparatus according to the present invention comprises a first electrode and a second electrode arranged at respective sides of an electrophoretic gel and each in close contact with one side of the electrophoretic gel. By using the first electrode and the second electrode each in close contact with one side of the electrophoretic gel, the horizontal electrophoresis apparatus enables electric current to flow across the entire cross section of the electrophoretic gel, and thus can prevent an electric current difference from occurring between the upper and lower ends of the electrophoretic gel and solve the problem of a reduction in the resolution of protein bands.

## Description

### [Technical Field]

The present invention relates to a horizontal electrophoresis apparatus, and more particularly, to a horizontal electrophoresis apparatus which uses a first electrode and a second electrode in close contact with one side of an electrophoretic gel to prevent an electric current difference between the top and bottom of the electrophoretic gel.

### [Background Art]

In the field of biotechnology, electrophoresis is generally performed for the analysis of nucleic acid molecules (DNA and RNA) or proteins.

Polyacrylamide gel electrophoresis (PAGE) is a method that uses a polyacrylamide gel for gel electrophoresis. The polyacrylamide gel is a porous material which is much denser than an agar gel and thus can be used to separate samples with small molecular weights and small size differences compared to the use of agar.

Meanwhile, sodium dodecyl sulfate-polyacrylamide gel electrophoresis (SDS-PAGE) is a method commonly used to spread proteins in a sample in order to efficiently detect a very small amount of proteins. The sodium dodecyl sulfite (SDS) binds to the proteins present in the sample to cause the proteins to lose their secondary, tertiary, and quaternary structures and become linear, and provides the proteins with a high density of negative charges such that the proteins are attracted toward the positive pole of the voltage applied during electrophoresis.

At this time, since the polyacrylamide gel is a dense porous material, the proteins having a linear structure and negatively charged by SDS can be classified according to the principle that the migration speed varies due to the size of the proteins as the electrophoresis is performed.

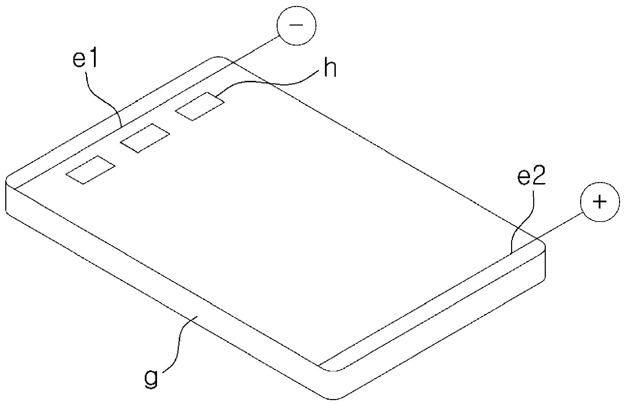

FIG. 1 shows a horizontal PAGE electrophoresis apparatus used for protein analysis. Referring to FIG. 1, conventionally, the horizontal PAGE electrophoresis for protein analysis is performed in a state where platinum wire electrodes e1 and e2 are placed on both ends of a gel g.

As such, according to the conventional horizontal PAGE electrophoresis apparatus, the platinum wire electrodes e1 and e2 are disposed on the top side of the gel g, and thus an electric current difference occurs between the top and bottom of the gel g.

That is, conventionally, more electric current flows through the top side of the gel g on which the platinum wire electrodes e1 and e2 are disposed, and due to this, when the protein sample migrates, the migration speed varies between the top and bottom of the gel g.

In other words, as the electric current difference occurs as described above, the migration at the top side of the gel g becomes faster, and thus when looking at the development of the protein sample, injected into the wells h, from the side, the protein bands are skewed.

Due to the above phenomenon, as a result of electrophoresis, the protein bands become thick, which results in a reduced resolution, and in particular, the resolution is significantly reduced for small proteins of medium or smaller size with a long migration distance.

### [Disclosure]

### [Technical Problem]

The present invention has been made to solve the above-mentioned conventional problems, and the object of the present invention is to provide a horizontal electrophoresis apparatus which uses a first electrode and a second electrode in close contact with one side of an electrophoretic gel to prevent an electric current difference between the top and bottom of the electrophoretic gel.

### [Technical Solution]

To achieve the above object, the present invention provides a horizontal electrophoresis apparatus comprising a first electrode and a second electrode disposed on both sides of an electrophoretic gel, the first electrode and the second electrode being in close contact with one side of the electrophoretic gel.

The first electrode and the second electrode may be in close contact with both lateral sides of the electrophoretic gel to face each other with the electrophoretic gel interposed therebetween.

The electrophoretic gel may be a polymer gel such as polyacrylamide or agar.

One of the first electrode and the second electrode may be electrically connected to a positive terminal of an external power supply, and the other may be electrically connected to a negative terminal of the external power supply.

Moreover, the cross-sections of the first electrode and the second electrode may surround the upper, lower, and lateral sides of the edge of the electrophoretic gel.

Moreover, each of the first electrode and the second electrode may comprise two electrodes; an upper electrode and a lower electrode in contact with the upper and lower sides of the electrophoretic gel.

The interval between the upper electrodes disposed on the upper side of the electrophoretic gel and the interval between the lower electrodes disposed on the lower side of the electrophoretic gel may be adjusted to control the amount of electric current flowing through the electrophoretic gel.

A wick may be disposed between each of the first electrode and the second electrode and the electrophoretic gel, and the wick may comprise an electrolyte.

### [Advantageous Effects]

The horizontal electrophoresis apparatus according to the present invention uses the first electrode and the second electrode in close contact with one side of the electrophoretic gel to allow the electric current to flow uniformly over the entire cross section of the electrophoretic gel, thereby preventing an electric current difference between the top and bottom of the electrophoretic gel and solving the problem that the resolution of the protein bands is reduced.

Moreover, the horizontal electrophoresis apparatus according to the present invention supplies sufficient electric current also to the lower side of the electrophoretic gel through the first electrode and the second electrode, which reduces the temperature difference between the top, middle and bottom of the electrophoretic gel, resulting in an improved quality of electrophoresis.

### [Description of Drawings]

FIG. 1 is a perspective view of a conventional horizontal electrophoresis apparatus.
FIG. 2 is a perspective view of a horizontal electrophoresis apparatus according to one embodiment of the present invention.
FIG. 3 is a cross-sectional view of a horizontal electrophoresis apparatus according to another embodiment of the present invention.
FIG. 4 shows the results of the electrophoresis performed using the electrophoresis apparatus of FIG. 3 and the results of the electrophoresis performed using a conventional horizontal electrophoresis apparatus.
FIG. 5 is a cross-sectional view of a horizontal electrophoresis apparatus according to another embodiment of the present invention.
FIG. 6 shows the amounts of electric current measured using upper electrodes only, using lower electrodes only, and using both the upper and lower electrodes, respectively.
FIG. 7 is a perspective view of an electrophoresis platform on which a horizontal electrophoresis apparatus according to another embodiment of the present invention is installed.
FIG. 8 is a perspective view showing the upper electrode of FIG. 7.
FIG. 9 is a perspective view showing the lower electrode of FIG. 7.
FIG. 10 shows the amounts of electric current flowing through the upper electrodes disposed on the upper side of the electrophoretic gel at different intervals.
FIG. 11 is a cross-sectional view showing a reduced interval between the lower electrodes of FIG. 5 and a cross-sectional view showing a reduced interval between the upper electrodes of FIG. 5.
FIG. 12 shows the results of the electrophoresis performed using the electrophoresis apparatus of FIG. 11(a).
FIG. 13 shows the results of the electrophoresis performed using the electrophoresis apparatus of FIG. 11(b).
FIG. 14 shows the results of the electrophoresis performed using the electrophoresis apparatus of FIG. 5.
FIG. 15 shows the results of the electrophoresis performed on an electrophoretic gel having a thickness of 1 mm using the electrophoresis apparatus of FIG. 5.

### [Mode for Invention]

Hereinafter, exemplary embodiments of the present invention will be described in detail with reference to the accompanying drawings. First of all, it should be noted that in giving reference numerals to elements of each drawing, like reference numerals refer to like elements even though like elements are shown in different drawings. It should be understood that although the exemplary embodiments of the present invention are described hereafter, the spirit of the present disclosure is not limited thereto and may be practiced by those skilled in the art.

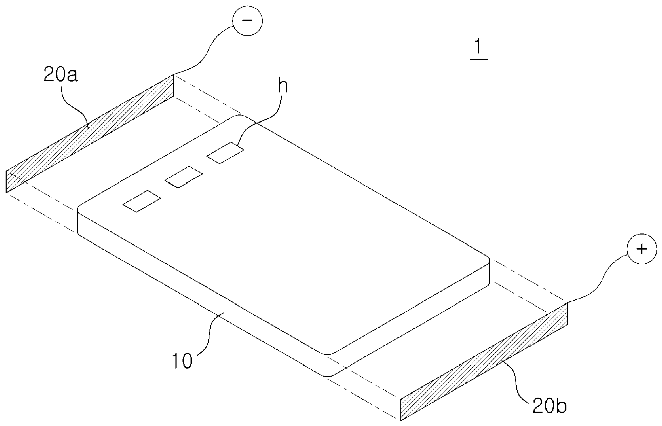

Hereinafter, a horizontal electrophoresis apparatus according to one embodiment of the present invention will be described with reference to FIG. 2.

Referring to FIG. 2, the horizontal electrophoresis apparatus 1 according to one embodiment of the present invention comprises a first electrode 20a and a second electrode 20b disposed on both sides of an electrophoretic gel 10.

Here, the electrophoretic gel 10 may be a polymer gel such as polyacrylamide, agar, etc. The polyacrylamide gel is a porous material which is much denser than an agar gel and thus can be used to separate samples with small molecular weights and small size differences compared to the use of agar. For example, the electrophoretic gel 10 may be a polyacrylamide gel (10% resolving gel), but is not limited thereto.

Moreover, the first electrode 20a and the second electrode 20b may be in close contact with one side of the electrophoretic gel 10.

As shown in FIG. 2, the first electrode 20a and the second electrode 20b may be in close contact with both lateral sides of the electrophoretic gel 10 to face each other with the electrophoretic gel 10 interposed therebetween.

Furthermore, the first electrode 20a and the second electrode 20b may be connected to an electric wire to be supplied with electricity from an external power supply. Here, one of the first electrode 20a and the second electrode 20b may be electrically connected to a positive terminal of the external power supply, and the other may be electrically connected to a negative terminal of the external power supply.

In addition, each of the first electrode 20a and the second electrode 20b may be a surface electrode made of a conductive material such as platinum, aluminum, etc.

For the electrophoresis process, when a protein sample is injected into wells h of the electrophoretic gel 10 and an electric current is supplied from an external power supply to the first electrode 20a and the second electrode 20b through the electric wire, the electric current flows through the electrophoretic gel 10 in close contact with the first electrode 20a and the second electrode 20b, which causes the protein sample to migrate, thereby causing protein separation and performing the electrophoresis.

As such, unlike the conventional horizontal electrophoresis apparatus as shown in FIG. 1, where the platinum wire electrodes e1 and e2 are placed on both ends of a gel g, the horizontal electrophoresis apparatus 1 according to the present invention uses the first electrode 20a and the second electrode 20b in close contact with both lateral sides of the electrophoretic gel 10 to prevent an electric current difference between the top and bottom of the electrophoretic gel 10.

That is, the electric current flows uniformly over the entire cross section of the electrophoretic gel 10 through the first electrode 20a and the second electrode 20b, and thus it is possible to solve the problem that a certain portion of the protein band becomes particularly thick as a result of the electrophoresis, which results in a reduced resolution.

Meanwhile, according to the horizontal electrophoresis apparatus 1 of the present invention, the position where the first electrode 20a and the second electrode 20b are in close contact with the electrophoretic gel 10 is not limited to both lateral sides as shown in FIG. 2, and the first electrode 20a and the second electrode 20b may be disposed in various positions, such as lateral and lower sides, lateral and upper sides, etc., resulting in an improved quality of electrophoresis.

In addition, when the first electrode 20a and the second electrode 20b of the present invention are used for two-dimensional polyacrylamide gel electrophoresis (2D PAGE), the electric current flows uniformly, which increases the number of protein spots, which serve as a standard for quality assessment of electrophoretic separation.

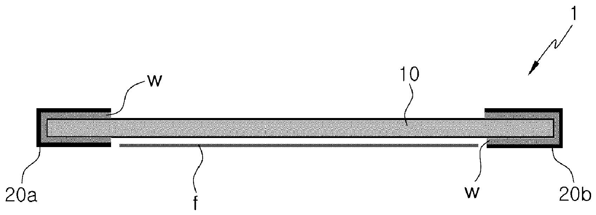

Next, the structure of a horizontal electrophoresis apparatus according to another embodiment of the present invention will be described with reference to FIG. 3. For convenience of description, the description of the same elements as those of the embodiment shown in FIG. 2 will be omitted, and the differences will be mainly described below.

According to the horizontal electrophoresis apparatus 1 of another embodiment of the present invention, the cross sections of the first electrode 20a and the second electrode 20b may surround the upper, lower, and lateral sides of the edge of the electrophoretic gel 10.

Here, a wick w may be disposed between each of the first electrode 20a and the second electrode 20b and the electrophoretic gel 10, and the wick w may comprise an electrolyte.

Specifically, the wick w may comprise a stack of filter papers and may be immersed in an electrolyte solution to allow the electric current to flow. With the wick w, the first and second electrodes 20a and 20b and the gel 10 do not directly contact one another, thereby preventing damage to the gel 10 and providing an electrolyte necessary for electrophoresis.

Moreover, a PVC film f may be disposed between the first electrode 20a and the second electrode 20b to support the lower side of the electrophoretic gel (10), whereby the electrophoretic gel (10) can be level between the first electrode 20a and the second electrode 20b.

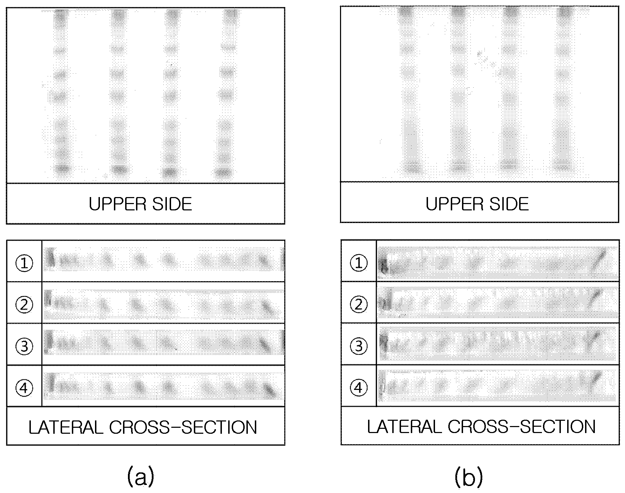

FIG. 4(a) shows the results of the electrophoresis performed using the first electrode 20a and the second electrode 20b of which the cross sections surround the upper, lower, and lateral sides of the edge of the electrophoretic gel 10 and which have a thickness of 3 mm.

At this time, a pre-stained protein marker was used as a sample, and the sample migrated from top to bottom in the case of the upper side image and from left to right in the case of the lateral cross-sectional image.

As shown in Fig. 4(a), according to the present invention, since the electric current flows uniformly over the entire cross section of the electrophoretic gel 10 through the first electrode 20a and the second electrode 20b, it can be seen that as the protein marker migrates during the electrophoresis, the respective protein bands are aligned nearly vertically and clearly distinguished from each other.

FIG. 4(b) shows the results of the electrophoresis performed at 40 mA using a conventional horizontal electrophoresis apparatus

As shown in Fig. 4(b), in the case of the conventional horizontal electrophoresis apparatus, the bands were shown to lie down while the protein migrated.

That is, since the conventional horizontal electrophoresis apparatus has a configuration in which the wire electrodes are disposed only on both top sides of the gel, the electric current strength on the upper side of the gel is higher than that on the lower side, which causes the protein bands to be particularly thick only in a specific region, resulting in a reduced resolution.

Meanwhile, heat generated in the electrophoretic gel 10 during the electrophoresis process may serve to interfere with the electrophoresis.

However, according to the horizontal electrophoresis apparatus 1 of the present invention, sufficient electric current can be also supplied to the lower side of the electrophoretic gel 10 through the first electrode 20a and the second electrode 20b of which the cross sections surround the upper, lower, and lateral sides of the edge of the electrophoretic gel 10.

Therefore, the present invention can not only prevent the protein bands from being skewed, but also reduce the temperature difference between the top, middle and bottom of the electrophoretic gel 10, thereby further improving the quality of electrophoresis.

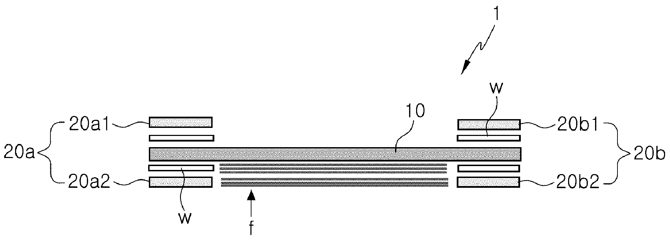

Next, the structure of a horizontal electrophoresis apparatus according to another embodiment of the present invention will be described with reference to FIG. 5. For convenience of description, the description of the same elements as those of the embodiment shown in FIG. 3 will be omitted, and the differences will be mainly described below.

According to the horizontal electrophoresis apparatus 1 of another embodiment of the present invention, each of the first electrode 20a and the second electrode 20b may comprise an upper electrode 20a1, 20b1 and a lower electrode 20a2, 20b2, which are in contact with the upper and lower sides of the electrophoretic gel 10, respectively.

Specifically, the first electrode 20a disposed on the left side of the electrophoretic gel 10 as shown in FIG. 5 may comprise an upper electrode 20a1 and a lower electrode 20a2, and the second electrode 20b disposed on the right side of the electrophoretic gel 10 as shown in FIG. 5 may comprise an upper electrode 20b1 and a lower electrode 20b2.

Here, the first electrode 20a and the second electrode 20b may be spaced about 1 cm and made of titanium coated with platinum.

As such, since each of the first electrode 20a and the second electrode 20b comprises the upper electrode 20a1, 20b1 and the lower electrode 20a2, 20b2, respectively, which are in contact with the upper and lower sides of the electrophoretic gel 10, the process of bringing the electrodes into contact with the electrophoretic gel 10 becomes easy.

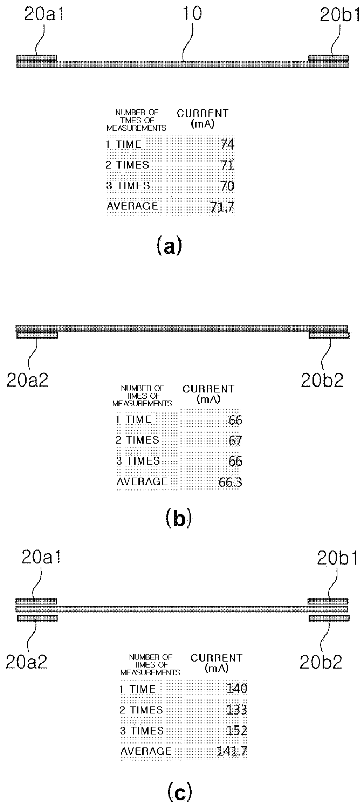

FIG. 6 shows the amounts of electric current measured using upper electrodes only, using lower electrodes only, and using both the upper and lower electrodes, respectively. At this time, the thickness of the electrophoretic gel 10 was 3 mm, the interval between the electrodes was about 6 cm, and the fixed voltage of the power supply was set to 50 V.

As a result of the measurement, it can be seen that, in the case where both the upper electrodes 20a1 and 20b1 and the lower electrodes 20a2 and 20b2 were used as shown in FIG. 6(c), the amount of applied electric current is higher, about two times, than that as shown in FIG. 6(a) and FIG. 6(b), respectively, and the amounts of applied electric current between the upper and lower electrodes are also similar.

Moreover, it is possible to precisely control the position or thickness of the protein bands by allowing different amounts of electric current to flow through the upper electrodes 20a1 and 20b1 and the lower electrodes 20a2 and 20b2 during the electrophoresis.

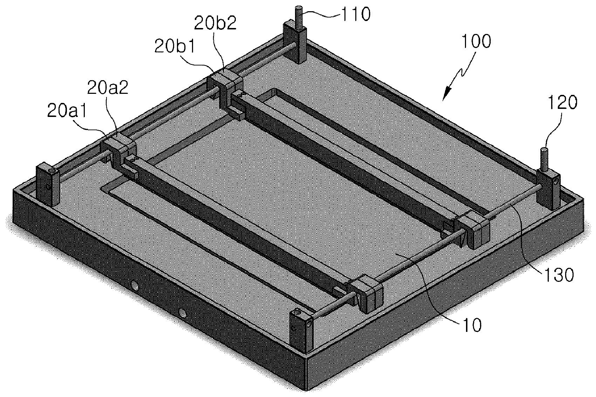

Specifically, referring to FIG. 7, an electrophoresis platform 100 may be used during the electrophoresis, and the electrophoresis platform 100 may comprise electrical terminals 110 and 120, which will be connected to a positive (+) terminal and a negative (-) terminal of a power supply (not shown), respectively, and a pair of rods 130 connected to the electrical terminals 110 and 120.

Here, the upper electrodes 20a1 and 20b1 and the lower electrodes 20a2 and 20b2 may be connected at their both ends to the rods 130 of the electrophoresis platform 100, respectively, to move their positions, and may be electrically connected to the power supply connected to the electrical terminals 110, 120 through the rods 130.

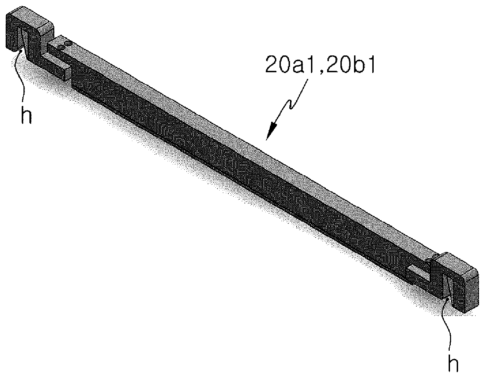

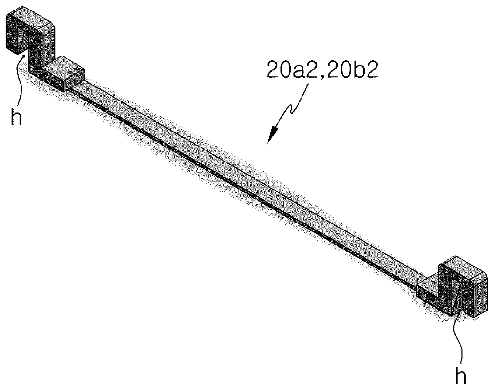

Referring to FIGS. 8 and 9, grooves h are formed at both ends of the upper electrodes 20a1 and 20b1 and the lower electrodes 20a2 and 20b2. Accordingly, the pair of rods 130 provided in the electrophoresis platform 100 can be inserted into the grooves h, and thus the upper electrode 20a1 and 20b1 and the lower electrodes 20a2 and 20b2 can move their positions along the longitudinal direction of the rods 130.

As such, according to the horizontal electrophoresis apparatus 1 of another embodiment of the present invention, it is possible to adjust the interval between the upper electrodes 20a1 and 20b1 disposed on the upper side of the electrophoretic gel 10 and the interval between the lower electrodes 20a2 and 20b2 disposed on the lower side of the electrophoretic gel 10.

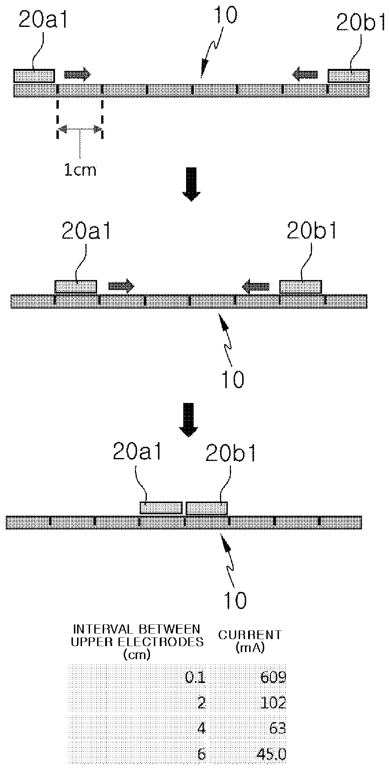

FIG. 10 shows the amounts of electric current flowing through the upper electrodes 20a1 and 20b1 disposed on the upper side of the electrophoretic gel 10 at different intervals. At this time, the fixed voltage of the power supply was set to 50 V.

As shown in FIG. 10, it can be seen that the amount of electric current increases as the interval between the upper electrodes 20a1 and 20b1 becomes narrower. That is, it can be seen that the amount of electric current flowing through the electrophoretic gel 10 can easily be controlled by adjusting the interval between the electrodes.

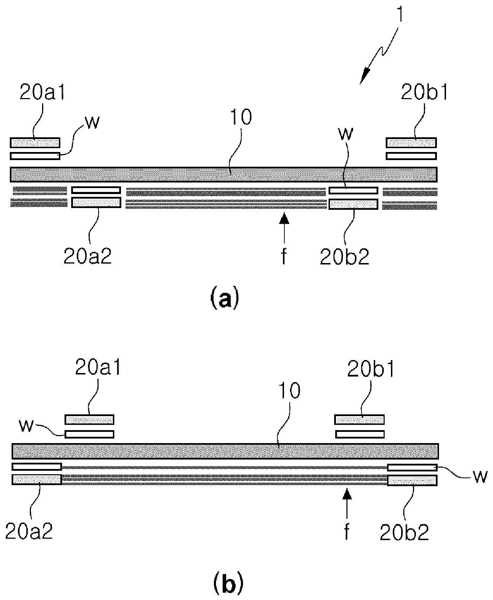

Referring to FIG. 11, the intervals between the upper electrodes 20a1 and 20b1 and the lower electrodes 20a2 and 20b2 may be independently adjusted.

That is, as shown in FIG. 11(a), the lower electrodes 20a2 and 20b2 can be moved so that the interval between them becomes narrower, and as shown in FIG. 11(b), the upper electrodes 20a1 and 20b1 can be moved so that the interval between them becomes narrower.

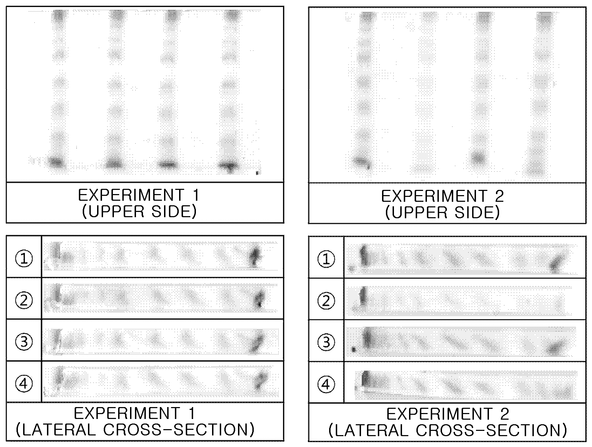

Referring to FIG. 12, when the interval between the lower electrodes 20a2 and 20b2 is narrowed as in FIG. 11(a), the distance between the lower electrodes 20a2 and 20b2 is relatively close, and thus the amount of electric current flowing along the lower side of the electrophoretic gel 10 increases, thereby allowing the proteins at the bottom of the gel 10 to migrate faster. As a result, it can be seen that the thickness of the bands observed at the top of the gel 10 becomes wider.

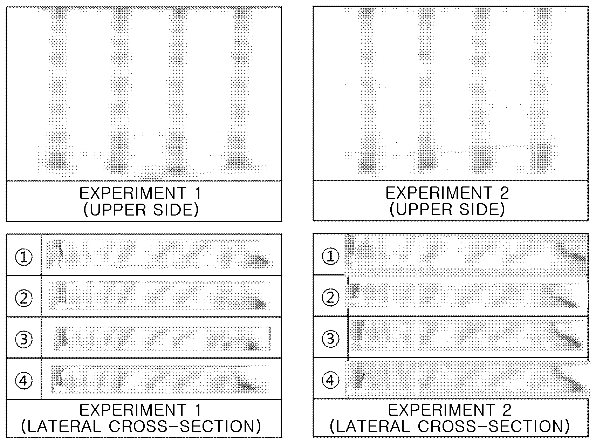

Referring to FIG. 13, when the interval between the upper electrodes 20a1 and 20b1 is narrowed as shown in FIG. 11(b), the distance between the upper electrodes 20a1 and 20b1 is relatively close, and thus the amount of electric current flowing along the upper side of the electrophoretic gel 10 increases, thereby allowing the proteins at the top of the gel 10 to migrate faster. As a result, it can be seen that the thickness of the bands observed at the bottom of the gel 10 becomes wider.

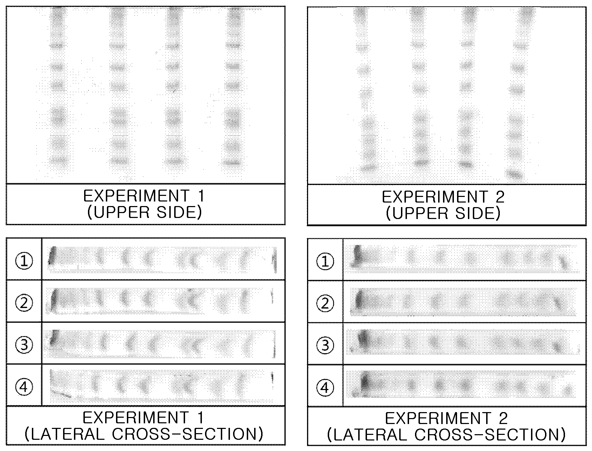

Referring to FIG. 14, when the interval between the upper electrodes 20a1 and 20b1 and the interval between the lower electrodes 20a2 and 20b2 are set to be the same, it can be seen that the protein bands are aligned nearly vertically.

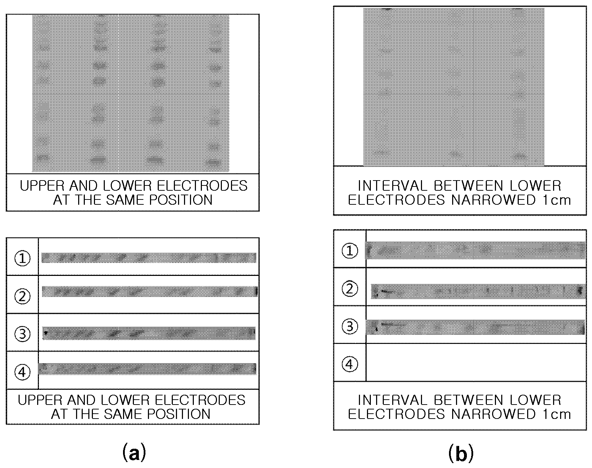

The above-mentioned results shown in FIGS. 12 to 14 were obtained using a gel 10 having a thickness of 3 mm, while FIG. 15 shows the results of the electrophoresis performed using a commonly used gel 10 having a thickness of 1 mm in a state where the upper electrodes 20a1 and 20b1 and the lower electrodes 20a2 and 20b2 are aligned in the same position.

Unlike the electrophoresis performed using the 3 mm thick gel 10, in the case where the 1 mm thick gel 10 was used, even if the upper electrodes 20a1 and 20b1 and the lower electrodes 20a2 and 20b2 are aligned in the same position, the protein bands were shown to be skewed in the direction of migration.

To solve this problem, the interval between the lower electrodes 20a2 and 20b2 was further narrowed by 1 cm to increase the amount of electric current flowing to the bottom of the gel 10, and as a result, the problem that the protein bands were skewed has been improved as shown in FIG. 15(b)

That is, it can be seen that the quality of electrophoresis can be improved by adjusting the interval between the upper electrodes 20a1 and 20b1 and the interval between the lower electrodes 20a2 and 20b2 to control the amounts of electric current at the top and bottom.

The invention has been described in detail with reference to preferred embodiments thereof. However, it will be appreciated by those skilled in the art that changes may be made in these embodiments without departing from the principles and spirit of the invention, the scope of which is defined in the appended claims and their equivalents.

## Claims

1. A horizontal electrophoresis apparatus comprising:
a first electrode and a second electrode disposed on both lateral sides of an electrophoretic gel, the first electrode and the second electrode being in close contact with one side of the electrophoretic gel.

2. The horizontal electrophoresis apparatus of claim 1, wherein the first electrode and the second electrode are in close contact with both lateral sides of the electrophoretic gel to face each other with the electrophoretic gel interposed therebetween.

3. The horizontal electrophoresis apparatus of claim 1,
wherein the electrophoretic gel is a polymer gel such as polyacrylamide or agar.

4. The horizontal electrophoresis apparatus of claim 1,
wherein one of the first electrode and the second electrode is electrically connected to a positive terminal of an external power supply, and the other is electrically connected to a negative terminal of the external power supply.

5. The horizontal electrophoresis apparatus of claim 1,
wherein the cross-sections of the first electrode and the second electrode surround the upper, lower, and lateral sides of the edge of the electrophoretic gel.

6. The horizontal electrophoresis apparatus of claim 1,
wherein each of the first electrode and the second electrode comprises an upper electrode and a lower electrode in contact with the upper and lower sides of the electrophoretic gel.

7. The horizontal electrophoresis apparatus of claim 6,
wherein the interval between the upper electrodes disposed on the upper side of the electrophoretic gel and the interval between the lower electrodes disposed on the lower side of the electrophoretic gel can be adjusted to control the amount of electric current flowing through the electrophoretic gel.

8. The horizontal electrophoresis apparatus of claim 1,
wherein a wick is disposed between each of the first electrode and the second electrode and the electrophoretic gel, and the wick comprises an electrolyte.
